# EUROPEAN PATENT APPLICATION

(11) **EP 2 444 813 A1**
(43) Date of publication of application: **25.04.2012**
(21) Application number: 10188168.8
(22) Date of filing: 20.10.2010
(51) Int. Cl.: G01N 33/68

(54) **S100B-based means and methods for diagnosing cerebral damages in acute inflammation**

(71) Applicant: Roche Diagnostics GmbH, 68305 Mannheim (DE); F. Hoffmann-La Roche AG, 4070 Basel (CH)
(72) Inventor: Hess, Georg, 55130 Mainz (DE); Horsch, Andrea, 68259 Mannheim (DE); Zdunek, Dietmar, 82327 Tutzing (DE)
(74) Representative: Dick, Alexander

(57) **Abstract**

The present invention relates to the field of diagnostic measures. Specifically, it relates to a method for diagnosing in a subject exhibiting a symptom of acute inflammation whether a cerebral damage is an ischemic cerebral damage or a non-ischemic cerebral damage comprising determining the amount of the biomarkers S100B and sFlt-1 in a sample of said subject and comparing said amount to reference amounts whereby it is diagnosed whether the cerebral damage is an ischemic cerebral damage or a non-ischemic cerebral damage. A method for diagnosing whether a subject exhibiting a symptom of acute inflammation is at increased risk of mortality comprising determining the amount of the biomarker S 100B in a sample of said subject and comparing said amount to a reference amount whereby it is diagnosed whether the subject is at increased risk of mortality, or not. Further encompassed are diagnostic devices and kits for carrying out the aforementioned methods.

## Description

The present invention relates to the field of diagnostic measures. Specifically, it relates to a method for diagnosing in a subject exhibiting a symptom of acute inflammation whether a cerebral damage is an ischemic cerebral damage or a non-ischemic cerebral damage comprising determining the amount of the biomarkers S100B and sFlt-1 in a sample of said subject and comparing said amount to reference amounts whereby it is diagnosed whether the cerebral damage is an ischemic cerebral damage or a non-ischemic cerebral damage. A method for diagnosing whether a subject exhibiting a symptom of acute inflammation is at increased risk of mortality comprising determining the amount of the biomarker S100B in a sample of said subject and comparing said amount to a reference amount whereby it is diagnosed whether the subject is at increased risk of mortality, or not. Further encompassed are diagnostic devices and kits for carrying out the aforementioned methods.

Fever is a frequent event and caused by infections, microbial toxins, mediators of inflammation and immune reactions. It is associated with the release of pyrogenic cytokines such as II 1, Il 6, TNF and interferons. These cytokines are responsible for the release of prostaglandin E 2 (PGE 2) in peripheral tissues.(Dinarello C.A., Porat R., in Principles of Internal Medicine 17. Ed p 117 ff). Most fevers are associated with self limited infections, such as common viral diseases and are, therefore, harmless. Thus, in most cases fever can be treated with antipyretics in order to achieve relief of symptoms. Antipyretics might, however, be contraindicated in patients with bacterial infections as they may mask inadequately treated bacterial infections. Specifically bacterial and fungal infections may become systemic and might progress to systemic inflammatory response syndrome (SIRS) which is characterized by hyper- or hypothermia, tachypnoe, tachycardia, leucocytosis or leucopenia.

SIRS associated with proven or suspected infection is called Sepsis. Sepsis can proceed to severe sepsis, septic shock, refractory septic shock or multi-organ dysfunction syndrome (MODS). The latter conditions are associated with organ dysfunction, e.g. the cardiovascular, the renal, the respiratory, the cerebral or the hematologic system. Attempts to improve survival in case of systemic infections, specifically sepsis have failed in the past. The major reason for the treatment failure was the clinically apparent development of organ dysfunction which could not be reversed by the treatment. Thus, it is important to identify individuals at risk of complications early to provide them with timely treatment (e.g. antibiotics). (Munford in Harrison Principles of Internal Medicine, 17, p.1695 ff). In fact, the time which elapsed until administration of an appropriate treatment is the primary determinant of mortality in patients with sepsis (Gaieski 2010, Crit Care Med 38 1045-1053).

Current methods to assess prognosis of a patient (preferably in the intensive care unit (ICU)) include the APACHE II Score as well as the SOFA Score. The APACHE II Score includes the assessment of temperature, mean blood pressure, heart rate, respiratory rate, arterial pH, oxygenation, serum sodium, serum potassium, hematocrit and white blood cell count. In addition age, chronic health status and the Glasgow coma Score is considered. Various studies indicate that the APACHE II Score is useful in predicting survival, survival rates have been shown to be higher in postoperative cases at the same APACHE II Score level. The APACHE II scoring system is not restricted to the ICU but can also be performed in the emergency room, this system is however time consuming as it cannot differentiate between different organ failures (Kress J.P., Hall J.B. in Harrison Principles of Internal Medicine p 1673 ff).

An alternative to the APACHE II Scoring system is the SOFA Score. The SOFA Score (Sepsis related organ failure Assessment) includes the determination of oxygenation (paO2/Fi2 (mmHg), platelet counts, bilirubin, blood pressure, Glasgow coma scale as well as kidney function (creatinine and urine volume/day) (Vincent et al, Intensive Care Med 1996, 22: 707 - 10). The SOFA Scoring System is also associated with prognosis of survival.

Disadvantage of both systems are that they are laborious and time consuming and might not pick up early changes/deterioration in organ function, which is of importance in the early recognition of complications and for treatment decisions (see above) or for the identification of patients at increased risk of complications (see above).

A severe organ complication in the context of acute inflammation is cerebral damage. The cerebral damage may result from either a damage of the blood brain barrier caused by the ongoing systemic infection. A damage of the blood brain barrier will allow for entering of endogenous and exogenous neurotoxic compounds found in the blood into the brain. These compounds will subsequently cause cell death in the brain resulting in severe neurological disorders. Moreover, as a consequence of acute inflammation cerebral damage arising from ischemia may occur. Circulation disorders which frequently occur during acute inflammation can cause ischemia in the central nervous system. As a result of the ischemia, cell death occurs in the affected areas of the central nervous system which in turn results in severe neurological disorders.

S100B is a 21 kDa calcium binding acidic dimeric protein that constitutes a major component of the cytosol of glial cells and is released into the peripheral circulation after cerebral damage (Bertsch 2001, Clin Chem Lab Med. 39: 319 - 323). Damage of the blood brain barrier also in the context of sepsis or glia damage during trauma have been reported to be accompanied by an increase in S100B, a member of the S100 protein family of neural proteins, in blood (Larsson 2005, Anesth Analg 101: 1465-1469; Hsu 2008, Pediatr Crit Care Med 9(3): 337-339; Stocchetti 2005, Trauma 101: 1463-1464).It has been shown to be also released into the circulation after ischemic cerebral stroke (Bertsch loc cit.) 4 to 72 hours after the ischemic event. S 100 B has also been detected in samples collects for the determination of procacitonin and with the clinical diagnosis of sepsis (EP 1 318 403 B 1). S100B has also been found in patients with trauma (Andersen 2001, Neurosurgery 1255 - 58, Unden 2005, J Trauma 58: 59 - 61), even in the absence of traumatic brain injury indicating that S100B does not exclusively originate from brain (Stocchetti loc cit.). Moreover, S100B has been detected in serum after liver injury associated with ligation even before reperfusion. Increased S100B concentrations have also been detected after femoral fracture without hemorrhagic shock. Increased levels of S100B were also found after myocardial infarction (Redl 2008, Pediatr. Crit Care Med. 9: 337 338). These data stress the fact that the release of S100B is not brain specific and appears to be related to ischemic processes.(Redl loc cit.).

It is desirable to identify patients presenting, e.g., to an emergency unit with a symptom of acute inflammation which indeed exhibit cerebral damage. Moreover, it is decisive for a therapy to distinguish between ischemic and non-ischemic cerebral damage since different therapeutic measures must be immediately applied in order to prevent the severe neurological consequences of cerebral damage as early as possible.

The technical problem underlying the present invention can be seen as the provision of means and methods for reliably and efficiently diagnosing in a subject exhibiting a symptom of acute inflammation whether a cerebral damage is an ischemic cerebral damage or a non-ischemic cerebral damage. The technical problem is solved by the embodiments characterized in the claims and herein below.

The present invention relates to a method for diagnosing in a subject exhibiting a symptom of acute inflammation whether a cerebral damage is an ischemic cerebral damage or a non-ischemic cerebral damage comprising:
a) determining the amount of the biomarkers S100B and sFlt-1 in a sample of said subject; and
b) comparing said amount to reference amounts whereby it is diagnosed whether the cerebral damage is an ischemic cerebral damage or a non-ischemic cerebral damage.

Preferably, it is diagnosed whether a cerebral damage is an ischemic cerebral damage or a non-ischemic cerebral damage by carrying out the further step of c) diagnosing whether a cerebral damage is an ischemic cerebral damage or a non-ischemic cerebral damage based on the result of the comparison carried out in step b).

The method of the present invention, preferably, is an ex vivo method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination in step (a) or a computer-implemented comparison and/or diagnosis based on said comparison in step (b).

The term "symptom of acute inflammation" as used herein refers to at least one symptom selected from a) white blood cell count (WBC) > about 12,000/ µ/L or < about 4000/µ/L, b) body temperature > about 38°C or < about 36°C, c) heart rate > about 90 beats/minute, d) a respiratory rate > about 20 breaths/minute or a partial pressure of CO2 of less than about 32 mm Hg, and e) more than 10% immature white blood cells among the counted white blood cells. Preferably, the term relates to fever (hyperthermia), i.e. a body temperature higher than about 38°C. Fever is, more preferably, characterized by a body temperature measured (i) in the rectum of at least 37.5-38.3°C (100-101°F), (ii) in the mouth (oral) of at least 37.7°C (99.9°F) or (iii) in the arm (axillary) or in the ear (otic) of at least 37.2°C (99.0°F). How to measure whether a subject exhibits fever is well known in the art.

The term "acute imflammation" refers to an acute systemic inflammation response in the subject. Said acute systemic inflammation may result from an infectious cause such as an expanding local infection which cannot be efficiently ameliorated due to an impaired function of the immune system in the subject or may be elicited by a non-infectious cause including trauma, bums, pancreatitis, ischemia and/or hemorrhage. Accordingly, acute systemic inflammation is characterized by a systemically reduced overall immune defense in the subject. The acute systemic inflammation may progress into the even more severe systemic inflammatory response syndrome (SIRS). SIRS as used in accordance with the present invention, preferably, encompasses SIRS as defined on the ACCP/SCCM Consensus Conference Definitions (1992/2003) (see e.g. American College of Chest Physicians/Society of Critical Care Medicine Consensus Conference: definitions for sepsis and organ failure and guidelines for the use of innovative therapies in sepsis. Crit Care Med. 1992 Jun;20(6):864-74)). Preferably, a patient is considered to suffer from SIRS, if the patient displays at least 2 symptoms of the following symptoms a) to e): a) white blood cell count (WBC) > about 12,000/ µ/L or < about 4000/µ/L, b) body temperature > about 38°C or < about 36°C, c) heart rate > about 90 beats/minute, d) a respiratory rate > about 20 breaths/minute or a partial pressure of CO2 of less than about 32 mm Hg, and e) more than 10% immature white blood cells among the counted white blood cells. The white blood cell count is usually determined by automated counting devices. As far as children are concerned, the consensus criteria for diagnosing SIRS in a child are disclosed in Goldstein et al. (Goldstein 2005, Pediatr. Crit Care Med 2005, 6(1),2- 8; see in particular Tables 2 and 3). An asymptomatic child patient in the sense of the present invention is a patient displaying less than 2, preferably less than 1 symptom of the ones described in Goldstein et al. (supra) which is incorporated herein by reference. Furthermore, in cases where SIRS may develop into sepsis if said disorder is accompanied by a proven or suspected microbial etiology, e.g., a systemic infection. An infection in the sense of the present invention preferably is a viral, fungus or bacterial infection, preferably a bacterial infection associated with bacteria selected from E coli, staphylococcus aureus, Klebsiella pneumoniae, Streptococci or Pseudomonas aeroginosa. The infection may as well be an infection by a fungus selected from Candida albicans, Candida tropicalis or Aspergillus fumigatus. An infection is diagnosed on the basis of assays and criteria generally known to the physician. Preferably, the infection is diagnosed on the basis of a bacterial culture assay, e.g., a culture medium inoculated with a sample from the patient, or based on molecular diagnostic methods. A fungus infection may, for example, be determined based on the generally known test assays such as Septifast. Sepsis may furthermore be determined by the aforementioned criteria for SIRS and at least two of the following criteria in addition: a) presence of leucocytes in a physiologically sterile body fluid; b) peritonitis or perforated viscus; c) pneumonia with focal opacification or petechiae, purpura, or purpura fulminans; d) bacteriemia. Further symptoms or particularly severe complications accompanying SIRS or sepsis are described in standard text books of medicine such as Stadmen or Pschyrembl. More preferably, the acute inflammation as used herein refers to SIRS and/or sepsis.

The term "cerebral damage" as used herein refers to tissue damage of the central nervous system and, in particular, the brain or certain associated tissues in this context such as the blood brain barrier, caused by endogenous influences. Preferably, the term refers to damage of neurons in the central nervous system. Tissue damage in the central nervous system can occur as a result of exogenous influences such as traumatic influences or, frequently, as an organ complication elicited by acute inflammation, i.e. endogenous influences. Cerebral damage as an organ complication, in particular, in the context of acute inflammation may occur as ischemic or non-ischemic cerebral damage. Cerebral damage may be ischemic cerebral damage. Said ischemic cerebral damage includes the damage caused by ischemic conditions in the central nervous system. As a consequence of the ischemia, cell death may result in the affected brain areas. This usually leads to severe neurological disorders. Cerebral damage can also be non-ischemic cerebral damage. Such a non-ischemic cerebral damage is, preferably, accompanied by an impaired function of the blood brain barrier, i.e. by damage of the said barrier. As a result of the damage of the blood brain barrier, the central nervous system is exposed to various exogenous and endogenous neurotoxic compounds which are present in the blood. As a consequence of the said exposure, cell death will arise as well.

The term "subject" as used herein relates to animals, preferably mammals, and, more preferably, humans. The subject according to the present invention shall exhibit a symptom of acute inflammation as described elsewhere herein. More preferably, said subject does or did not suffer from trauma, acute cardiovascular events, renal failure, and/or apparent malignancy, in particular, melanoma. Furthermore, the subject is, preferably, a subject presenting to an emergency unit.

The term "diagnosing" as used herein means assessing whether a subject exhibiting a symptom of acute inflammation has a non-ischemic cerebral damage or an ischemic cerebral damage. As will be understood by those skilled in the art, such an assessment is usually not intended to be correct for 100% of the subjects to be diagnosed. The term, however, requires that the assessment is correct for a statistically significant portion of the subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein.

The term "S100B or S100 calcium binding protein B" as used herein refers to a protein of the so called 5100 family of proteins. The S-100 protein family is a family of low molecular weight vertebrate proteins which are characterized by two calcium binding sites of helix-loop-helix ("EF-hand type") conformation. There are at least 21 different types of S 100 proteins (Marenholz 2004, Biochem Biophys Res Commun 322(4): 1111-1122. The name originates from the property of the members of the protein family to be 100% soluble in ammonium sulfate at neutral pH. S 100B is a glia specific S 100 protein and pivotally expressed in astrocytes. S100B is supposed to function in neurite extension, proliferation of melanoma cells, stimulation of Ca2+ fluxes, inhibition of PKC-mediated phosphorylation, astrocytosis and axonal proliferation, and inhibition of microtubule assembly. In the developing central nervous system, the protein acts as a neurotrophic factor and neuronal survival protein. Purification and structure of human S100B as well as assays for determining the biological and/or immunological properties have been reported in Jensen 1985, J. Neurochem. 45 (3): 700-705 and Baudier 1984, Biochim. Biophys. Acta 790 (2): 164-173 (see also NP_006263.2) Orthologs from mice have been reported as well (see NP_033141.1). Preferably, S100B refers to human S100B as described above. Furthermore, the term encompasses variants of said human S100B. Such variants have at least the same essential biological and immunological properties as the specific S100B polypeptide. Variants are deemed to share the same essential biological and immunological properties if they are detectable by the same specific assays referred to in this specification, e.g., by ELISA assays using polyclonal or monoclonal antibodies specifically recognizing the said S100B polypeptides. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific S100B polypeptides. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments of the specific S 100B polypeptides or the aforementioned types of variants as long as these fragments have the essential immunological and biological properties as referred to above. Such fragments may be, e.g., degradation products or splice variants of the s100B polypeptides. Further included are variants which differ due to posttranslational modifications such as phosphorylation or myristylation.

The term "soluble Flt-1" or "sFlt-1" as used herein refers to polypeptide which is a soluble form of the VEGF receptor Flt1. It was identified in conditioned culture medium of human umbilical vein endothelial cells. The endogenous soluble Flt1 (sFlt1) receptor is chromatographically and immunologically similar to recombinant human sFlt1 and binds [1251] VEGF with a comparable high affinity. Human sFlt1 is shown to form a VEGF-stabilized complex with the extracellular domain of KDR/Flk-I in vitro. Preferably, sFlt1 refers to human sFlt1 as describe in Kendall 1996, Biochem Biophs Res Commun 226(2): 324-328 (for amino acid sequences, see, e.g., also P17948, GI: 125361 for human and BAA24499.1, GI: 2809071 for mouse sFlt-1). The term also encompasses variants of the aforementioned human sFlt-1 polypeptides. Such variants have at least the same essential biological and immunological properties as the aforementioned sFlt-1 polypeptide. In particular, they share the same essential biological and immunological properties if they are detectable by the same specific assays referred to in this specification, e.g., by ELISA assays using polyclonal or monoclonal antibodies specifically recognizing the said sFlt-1 polypeptides. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific sFlt-1 polypeptide, preferably over the entire length of the human sFlt-1, respectively. The degree of identity between two amino acid sequences can be determined by algorithms well known in the art and described elsewhere herein. Variants referred to above may be allelic variants or any other species specific homologs, paralogs, or orthologs. Moreover, the variants referred to herein include fragments or subunits of the specific sFlt-1 polypeptides or the aforementioned types of variants as long as these fragments have the essential immunological and biological properties as referred to above. Such fragments may be, e.g., degradation products of the sFlt-1 polypeptides. Further included are variants which differ due to posttranslational modifications such as phosphorylation or myristylation.

Determining the amount of a peptide or polypeptide referred to in this specification relates to measuring the amount or concentration, preferably, semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay and methods which may utilize labeled molecules in various sandwich, competition, or other assay formats. Such assays are, preferably, based on detection agents such as antibodies which specifically recognize the peptide or polypeptide to be determined. The detection agents shall be either directly or indirectly capable of generating a signal indicating the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprises the steps of (a) contacting the peptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Further suitable techniques for the determination of a polypeptide or peptide are described in the following.

First, binding of a ligand may be measured directly, e.g. by NMR or surface plasmon resonance. Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the amount of the peptide or polypeptide comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison of the amount determined in step a) and the reference amount, it is possible to assess whether a cerebral damage is a ischemic or non-ischemic cerebral damage. Therefore, the reference amount is to be chosen so that either a difference or a similarity in the compared amounts allows identifying those test subjects which belong into the group of subjects exhibiting a symptom of acute inflammation having either a ischemic or non-ischemic cerebral damage. The method allows either excluding (rule-out) or identifying (rule-in) subject as a subject suffering from (i) a ischemic cerebral damage or (ii) a non-ischemic cerebral damage.

The term "reference amount" as used herein refers to an amount which allows for allocation of a subject into either the group of subjects exhibiting a non-ischemic cerebral damage or the group of subjects exhibiting ischemic cerebral damage. Such a reference amount can be a threshold amount which separates these groups from each other. Moreover, it will be understood that the method of the present invention is based on the application of a combination of different markers providing different information required for the diagnosis. Accordingly, the reference amount for S100B shall be an amount which allows for allocation of a subject into a group of subjects exhibiting cerebral damage or into a group of subjects which do not exhibit cerebral damage. A suitable threshold amount separating the two groups can be calculated without further ado by the statistical tests referred to herein elsewhere based on amounts of S100B from either a subject or group of subjects known to suffer from cerebral damage or a subject or group of subjects known not to suffer from cerebral damage. The further allocation into the group of subjects exhibiting non-ischemic cerebral damage or the group of subjects exhibiting ischemic cerebral damage is achieved according to the method of the present invention based on a sFlt-1 or HGF reference amount or both. A suitable threshold amount which separates these groups can be calculated without further ado by the statistical tests referred to herein elsewhere based on amounts of such biomarkers from either a subject or group of subjects known to suffer from ischemic cerebral damage or a subject or group of subjects known to suffer from non-ischemic cerebral damage. Similarly, a suitable threshold amount which separates these groups can be calculated without further ado by the statistical tests referred to herein elsewhere based on amounts of such biomarkers from either a subject or group of subjects known not to suffer from ischemic cerebral damage or a subject or group of subjects known not to suffer from non-ischemic cerebral damage.

Preferably, said reference amount is derived from a subject or a group of subjects known to suffer from non-ischemic cerebral damage and wherein an essentially identical or increased amount of the biomarker S100B in combination with an essentially identical or decreased amount for the biomarker sFlt-1 is indicative for a subject having a non-ischemic cerebral damage and wherein an essentially identical or increased amount of the biomarker S 100B in combination with an increased amount for the biomarker sFlt-1 is indicative for a subject having a ischemic cerebral damage.

Also preferably, said reference amount is derived from a subject or a group of subjects known to suffer from ischemic cerebral damage and wherein an essentially identical or increased amount of the biomarker S 100B in combination with an essentially identical or increased amount for the biomarker sFlt-1 is indicative for a subject having a ischemic cerebral damage and wherein an essentially identical or increased amount of the biomarker S100B in combination with a decreased amount for the biomarker sFlt-1 is indicative for a subject having a non-ischemic cerebral damage.

The reference amount applicable for an individual subject may vary depending on various physiological parameters such as age, gender, or subpopulation, as well as on the means used for the determination of the polypeptide or peptide referred to herein. A suitable reference amount may be determined from a reference sample to be analyzed together, i.e. simultaneously or subsequently, with the test sample.

A preferred reference amount indicating ischemic or non-ischemic cerebral damage is an amount of S 100B of about 0.061 pg/ml to about 0.199 pg/ml and, more preferably, about 0.091 pg/ml. A test amount being essentially identical or increased shall be indicative for a cerebral damage while an decreased amount shall be indicative for a lack of cerebral damage. Also, a preferred reference amount indicating ischemic or non-ischemic cerebral damage is an amount of S 100B of about 0.045 pg/ml to about 0.065 pg/ml and, more preferably, about 0.055 pg/ml. A test amount being essentially identical or decreased shall be indicative for a lack of cerebral damage while an increased amount shall be indicative for a cerebral damage.

A preferred reference amount indicating ischemic cerebral damage is an amount of sFlt-1 of about 42 pg/ml to about 71 pg/ml or of about 75 pg/ml to about 124 pg/ml and, more preferably, about 52 pg/ml or about 94 pg/ml. A test amount being essentially identical or decreased shall be indicative for a non-ischemic cerebral damage while an increased amount shall be indicative for a ischemic cerebral damage. Also, a preferred reference amount indicating ischemic cerebral damage is an amount of sFlt-1 of about 101.03 pg/ml to about 193.76 pg/ml and, more preferably, about 128.64 pg/ml. A test amount being essentially identical or increased shall be indicative for a ischemic cerebral damage while a decreased amount shall be indicative for a non-ischemic cerebral damage.

Reference amounts can, in principle, be calculated for a cohort of subjects as specified above based on the average or mean values for a given biomarker by applying standard statistically methods. In particular, accuracy of a test such as a method aiming to diagnose an event, or not, is best described by its receiver-operating characteristics (ROC) (see especially Zweig 1993, Clin. Chem. 39:561-577). The ROC graph is a plot of all of the sensitivity/specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1-specificity for the complete range of thresholds suitable for making a distinction. On the y-axis is sensitivity, or the true-positive fraction, which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the aforementioned method of the present invention can be, preferably, a threshold or cut off amount and can be generated, preferably, by establishing a ROC for said cohort as described above and deriving a threshold amount therefrom. Dependent on a desired sensitivity and specificity for a diagnostic method, the ROC plot allows deriving suitable thresholds. It will be understood that an optimal sensitivity is desired for excluding a subject for being at increased risk (i.e. a rule out) whereas an optimal specificity is envisaged for a subject to be assessed as being at an increased risk (i.e. a rule in).

Also preferably, the median values determined in the Tables of the accompanying Examples may be also used as a basis for establishing thresholds. Preferably, said threshold for S 100B which indicates cerebral damage, or not, is within the range of the 50^{th} percentile to the 75^{th} percentile of healthy subjects, i.e. about 0.055 pg/ml to about 0.065 pg/ml, more preferably, it is within the 25^{th} percentile to the 50^{th} percentile of subjects exhibiting a symptom of acute inflammation, i.e. about the 0.061 pg/ml to about 0.091 pg/ml. In this case, a test amount being larger than the threshold shall be indicative for cerebral damage while a test amount being essentially identical or below the threshold shall be indicative for a lack of a cerebral damage.

Preferably, said threshold for sFlt-1 which indicates ischemic cerebral damage, or not, is within the range of the 50^{th} percentile to the 75^{th} percentile of healthy subjects or subjects suffering from coronary artery disease, i.e. about 52 pg/ml to about 71 pg/ml or about 94 pg/ml to about 124 pg/ml, more preferably, it is within the range of the 25^{th} percentile to the 50^{th} percentile of subjects exhibiting a symptom of acute inflammation, i.e. about the 101.03 pg/ml to about 128.64. A test amount being larger than the threshold shall be indicative for ischemic cerebral damage while a test amount being essentially identical or below the threshold shall be indicative for a non-ischemic cerebral damage.

The term "about" in the context of the present invention means +/- 20%, +/- 10%, +/- 5%, +/-2 % or +/- 1% from the said values. This also takes into account usual deviations caused by measurement techniques, statistics and the like.

In principle, the present invention also relates to a method for diagnosing in a subject exhibiting a symptom of acute inflammation whether a cerebral damage is an ischemic or non-ischemic cerebral damage, said method comprising diagnosing whether a cerebral damage is a ischemic or non-ischemic damage based on a comparison of the amount of the biomarker S100B in combination with sFlt-1 in a sample of said subject to reference amounts.

Advantageously, it has been found in the studies underlying the present invention that the amount a combination of the biomarker S 100B indicating cerebral damage and the ischemia biomarkers sFlt-1 in a body fluid such as blood, plasma or serum allow for diagnosing whether a cerebral damage occurred in the subject, or not, and whether said cerebral damage is an ischemic or non-ischemic cerebral damage. The diagnostic rational can be summarized in general as follows:

| S100B: | sFlt-1: | Interpretation: |
|---|---|---|
| Normal | Normal | No cerebral damage, no ischemia |
| Elevated | Normal | Cerebral damage without ischemia (e.g., toxic cerebral damage) |
| Elevated | Elevated | Ischemic cerebral damage |
| Normal | Elevated | Ischemic events without cerebral damage |

This discrimination between the ischemic and non-ischemic cerebral damage is important for the further therapeutic steps envisaged. For example, a non-ischemic cerebral damage including damage of the blood-brain-barrier usually requires an immediate detoxification in order to limit potential further damages to the central nervous system and in particular the brain. On the other hand, ischemic damage requires immediate measures restoring the circulation in order to prevent cerebral damage arising from ischemia in the central nervous system. Thanks to the present invention, it is possible to more reliably diagnose and treat subjects in particular in emergency situation where a fast and reliable diagnosis is required. Moreover, the time consuming, expensive and cumbersome diagnostic measures can be avoided when applying the biomarker-based method of the invention as an aid for diagnosis. As acute inflammation represents a pathological process involving different organs and as brain damage is a severe complication of acute inflammation, the present invention shall improve the handling and treatment of patients suffering from acute inflammation.

It is to be understood that the definitions and explanations of the terms made above and below apply accordingly for all embodiments described in this specification and the accompanying claims.

In a preferred embodiment of the method of the present invention, said method further comprises recommending a therapy for the cerebral damage.

The term "recommending" as used herein means establishing a proposal for a therapy which could be applied to the subject. However, it is to be understood that applying the actual therapy whatsoever is not comprised by the term. The therapy to be recommended depends on the outcome of the diagnosis provided by the method of the present invention.

Preferably, said therapy of the cerebral damage is a circulation stabilizing therapy if the cerebral damage is ischemic cerebral damage. Preferred circulation stabilizing therapies are volume supplementation, vassopressor-based therapies such as dobutamine or vasopressin administration, oxygen supply including artificial ventilation, hydrocortisone administration and transfusions, and/or administration of diuretics such as furosemid. When said therapy of the cerebral damage is a detoxification therapy, i.e. if the cerebral damage is non-ischemic cerebral damage, the said therapy, preferably, is or involves dialysis or other absorption-based detoxification measures.

The present invention also pertains to a method for determining whether a therapy of non-ischemic or ischemic cerebral damage is successful in a subject exhibiting a symptom of acute inflammation comprising:
a) determining the amount of S100B and sFlt-1 in a first sample of said subject which has been obtained prior to the onset of the therapy and in a second sample which has been obtained after the onset of the therapy; and
b) comparing the amounts for the biomarkers determined in the first with the amounts determined in the second sample, whereby (i) in the case of non-ischemic cerebral damage a reduced amount of the biomarker S100B and an essentially identical amount of sFlt-1 found in the second sample compared to the first sample is indicative for a successful therapy of said non-ischemic cerebral damage and whereby (ii) in the case of ischemic cerebral damage a reduced amount of the biomarker S 100B and a reduced amount of sFlt-1 found in the second sample compared to the first sample is indicative for a successful therapy of said ischemic cerebral damage.

Thus, the method of the present invention also allows for monitoring the success of a therapy against cerebral damage and, thus, supports the hospital care management. A successful therapy as meant herein is accompanied by a decrease of the amount of S 100B found in a sample after the onset of the therapy, i.e. a second sample as referred to above. Suitable therapies which can be applied are described elsewhere herein. Depending on whether the cerebral damage is ischemic or non-ischemic cerebral damage, the amount of sFlt-1 shall also decrease.

The present invention also relates to a method for diagnosing whether a subject exhibiting a symptom of acute inflammation is at increased risk of mortality comprising:
a) determining the amount of the biomarker S100B in a sample of said subject; and
b) comparing said amount to a reference amount whereby it is diagnosed whether the subject is at increased risk of mortality, or not.

The term "increased risk of mortality" as used herein refers to statistically significant increase in the likelihood for mortality for a subject compared to the average or mean mortality likelihood within a given cohort of subjects, e.g., subjects suffering from acute inflammation in the present case. Whether there is a statistically significant increase of the risk can be determined by the skilled artisan by applying statistical tests recited elsewhere herein. The risk is preferably assessed for a predictive window. Said window is preferably 30 days or less, more preferably up to 4 weeks, 3 weeks, 2 weeks, one week, 3 days, 2 days or 1 day.

Suitable reference amounts can be obtained from samples of subjects known to be at increased risk of mortality. Identical amounts will than indicate whether the investigated subject is also at increased risk or not. The same applies mutatis mutandis for references from subjects known not be at increased risk of mortality.

Thus, said reference amount is, preferably, derived from a subject or a group of subjects exhibiting a symptom of acute inflammation which is known to be at increased risk of mortality and wherein an essentially identical or increased amount of said S100B is indicative for a subject being at increased risk of mortality and wherein a decreased amount of said S 100B is indicative for a subject being at normal risk of mortality.

Also preferably, said reference amount is derived from a subject or a group of subjects exhibiting a symptom of acute inflammation which is known to be at normal risk of mortality and wherein an essentially identical or decreased amount of said S100B is indicative for a subject being at normal risk of mortality and wherein a increased amount of said S100B is indicative for a subject being at increased risk of mortality.

A preferred reference amount is an amount of S 100B of about 0.06 to 0.19 ng/L and, more preferably, about 0.09 ng/L for survivors. A test amount being essentially identical or decreased shall be indicative for a normal risk of mortality while an increased amount shall be indicative for an increased risk of mortality. Also, a preferred reference amount is an amout of S100B of about 0.1 ng/L to about 0.73 ng/L and, more preferably, about 0.18 ng/L or more for non-survivors. A test amount being essentially identical or increased shall be indicative for an increased risk of mortality while a decreased amount shall be indicative for a normal risk of mortality.

Moreover, in a preferred embodiment of the aforementioned method for risk stratification, sFlt-1 can be also determined to strengthen the diagnosis. Thus, the preferred method comprises determining the amount of SFlt-1 in addition to S100B in the sample of the subject. The said amount of sFlt-1 determined in a test sample will then be compared to a reference amount whereby the diagnosis as to whether the subject is at increased risk of mortality, or not, will be strengthened.

A preferred reference amount is an amount of sFlt-1 of about 98 to about 185 pg/ml and, more preferably, about 127 pg/ml for survivors. A test amount being essentially identical or decreased shall be indicative for a normal risk of mortality while an increased amount shall be indicative for an increased risk of mortality. Also, a preferred reference amount is an amout of sFlt-1 of about 131 pg/ml to about 510 pg/ml and, more preferably, about 344 pg/ml or more for non-survivors. A test amount being essentially identical or increased shall be indicative for an increased risk of mortality while a decreased amount shall be indicative for a normal risk of mortality.

Advantageously, it has been found in the studies underlying the present invention that S100B serves as a biomarker for risk stratification in subjects exhibiting a symptom of acute inflammation. Accordingly, the method allows for a efficient risk stratification with respect to the mortality risk of subjects exhibiting said symptom and presenting in emergency units. Due to the risk stratification, further therapeutic measures such as monitoring and intensive care can be started at an earlier point which in case of a subject having an undesirable prognosis will help to improve said prognosis. The combined determination of S100B and sFlt-1 even strengthens the diagnosis.

In a further preferred embodiment of the method of the present invention, HGF, TGFß-1, BMP2 and/or BMP7 is determined in addition or, preferably, instead of S100B.

Further, the present invention relates to the use of the biomarker S100B and sFlt-1 or detection agents which specifically bind thereto in a sample of a subject exhibiting a symptom of acute inflammation for diagnosing whether a cerebral damage is an ischemic cerebral damage or a non-ischemic cerebral damage.

The term "detection agent" as used herein refers to an agent which is capable of specifically recognizing and binding to one of the biomarkers referred to above when present in a sample. Moreover, the said agent shall allow for direct or indirect detection of the complex formed by the said agent and the biomarker. Direct detection can be achieved by including into the agent a detectable label. Indirect labelling may be achieved by a further agent which specifically binds to the complex comprising the biomarker and the detection agent wherein the said further agent is than capable of generating a detectable signal. Suitable compounds which can be used as detection agents are well known in the art. Preferably, the detection agent is an antibody or aptamere which specifically binds to the biomarker.

Moreover, the present invention relates to the use of the biomarker S 100B or a detection agent that specifically binds thereto in a sample of a subject exhibiting a symptom of acute inflammation for diagnosing whether a subject exhibiting a symptom of acute inflammation is at increased risk of mortality.

The present invention also pertains to a device adapted for carrying out the method of the present invention for diagnosing whether a cerebral damage is an ischemic cerebral damage or a non-ischemic cerebral damage comprising
a) an analyzing unit comprising a detection agent which specifically binds to S100B and detection agents which specifically bind to sFlt-1, said unit being adapted for determining the amount of S100B and the amount of sFlt-1 in a sample of a subject exhibiting a symptom of acute inflammation; and
b) an evaluation unit for comparing the determined amounts with reference amounts whereby it can be diagnosed whether a cerebral damage is an ischemic cerebral damage or a non-ischemic cerebral damage, said unit comprising a database with reference amount values derived from a subject as defined above and a computer-implemented algorithm for carrying out a comparison as specified above.

The term "device" as used herein relates to a system comprising the aforementioned units operatively linked to each other as to allow the diagnosis according to the methods of the invention. Preferred detection agents which can be used for the analyzing unit are disclosed elsewhere herein. The analyzing unit, preferably, comprises said detection agents in immobilized form on a solid support which is to be contacted to the sample comprising the biomarkers the amount of which is to be determined. Moreover, the analyzing unit can also comprise a detector which determines the amount of detection agent which is specifically bound to the biomarker(s). The determined amount can be transmitted to the evaluation unit. Said evaluation unit comprises a data processing element, such as a computer, with an implemented algorithm for carrying out a comparison between the determined amount and a suitable reference. Suitable references can be derived from samples of subjects to be used for the generation of reference amounts as described elsewhere herein above. The results may be given as output of parametric diagnostic raw data, preferably, as absolute or relative amounts. It is to be understood that these data will need interpretation by the clinician. However, also envisage are expert system devices wherein the output comprises processed diagnostic raw data the interpretation of which does not require a specialized clinician.

Moreover, encompassed is also a device adapted for carrying out the method for diagnosing whether a subject exhibiting a symptom of acute inflammation is at increased risk of mortality comprising
a) an analyzing unit comprising a detection agent which specifically binds to S100B, said unit being adapted for determining the amount of S100B in a sample of a subject exhibiting a symptom of acute inflammation; and
b) an evaluation unit for comparing the determined amounts with reference amounts whereby it can be diagnosed whether a subject exhibiting a symptom of acute inflammation is at increased risk of mortality, said unit comprising a database with reference amount values derived from a subject as defined above and a computer-implemented algorithm for carrying out a comparison as specified above.

The present invention contemplates a kit adapted for carrying out the method of the present invention for diagnosing whether a cerebral damage is an ischemic cerebral damage or a non-ischemic cerebral damage, said kit comprising detection agents for the biomarker S100B and sFlt-1 as well as instructions for carrying out the said method.

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as a optical storage medium (e.g., a Compact Disc) or directly on a computer or data processing device. Moreover, the kit may, preferably, comprise standards for reference amounts as described elsewhere herein in detail.

A further kit adapted for carrying out the method for diagnosing whether a subject exhibiting a symptom of acute inflammation is at increased risk of mortality is contemplated by the present invention, said kit comprising a detection agent for the biomarker S 100B as well as instructions for carrying out the said method.

All references cited in this specification are herewith incorporated by reference with respect to their entire disclosure content and the disclosure content specifically mentioned in this specification.

### FIGURES

- Figure 1:: shows the association of S100B with the APACHE II Score in patients with acute inflammation.
- Figure 2:: shows the association of S100B with the SOFA Score in patients with acute inflammation.
- Figure 3:: shows the association of sFlt-1 with the APACHE II Score in patients with acute inflammation.
- Figure 4:: shows the association of sFlt-1 with the SOFA Score in patients with acute inflammation.
- Figure 5:: Correlation between S100B and sFlt-1 in patients with acute inflammation.

The invention will be illustrated by the following Examples which, however, shall not be construed as limiting the scope.

### EXAMPLES

### Example 1: Patients and determination of biomarkers

The following patient cohorts were investigated:
212 patients presenting to the emeregency department with a body temperature exceeding 38,5 degrees celsius, a heart rate above 90 beats per minute and a ventilatory frequency above 20 bpm were included into the study. Patients on dialysis or with impaired kidney function were excluded from the study, at presentation in none of the patients acute kidney injury was diagnosed. They were 102 females (mean age 65 years (range 21-91 years) and 110 males, mean age 62 years (range 18 -94 years). In all patients the APACHE II Score and the SOFA Score were recorded, in addition death at 30 days.

A control group consisted of 149 clinically healthy individuals ( n = 51 males, mean age 40 years (range 20 - 52 years) N = 97 females mean age 41 years (range 18-56 years) were included into the study, they had repeatedly normal blood pressure, a normal electrocardiogram, no diabetes and no history of cardiac disease or other disorders that would have put them at increased risk of cardiac disorders.

A control group consisted of 239 patients with symptomatic but stable heart failure, all patients had a kidney function within the normal range as measured by creatinine levels within normal. They were 186 males and 53 females, mean age 61.2. years, ischemic heart disease was present in 212 patients, all others had nonischemic heart disease.

The following methods and devices were used for determining the amount of the biomarkers referred to herein:
S100B, sFlt-1, NT-proBNP and GDF-15 were determined with sandwich immuno-assays using analyzers from Roche/Hitachi, Elecsys or COBAS e-series. The assays comprise two monoclonal antibodies specific for the respective peptide. The first of these iv biotinylated and the second one in labelled with a Tris(2,2'-bibyridyl)ruthemium (II)-complex. In a first incubation step both antibodies are incubated with the sample. A sandwich complex comprising the peptide to be determined and the two different antibodies is formed. In a next incubation step streptavidin-coated beads are added to this complex. The beads bind the sandwich complexes. The reaction mixture is then aspirated into a measuring cell where the beads are magnetically captured on the surface of the electrode. The application of a voltage then induces a chemiluminescent emission from the ruthenium complex which is measured by a photomultiplier. The emitted amount of light is dependent on the amount of sandwich complexes on the electrode. S100B amounts between 0.005 to 39 µg/L, sFlt-1 amounts between 10 to 85,000 pg/ml, GDF-15 amounts between 300 pg to 20,000 pg/ml, and NT-proBNP amounts between 2 pg/ml and 35,000 pg/ml can be measured.

Troponin T (hsTNT) was also determined using the aforementioned automatic analysers. The test followed the same test principles as described for NT-pro BNP. The high sensitivity Troponin T test used in this study has a sensitivity of 2 pg/ml and can be used on ELECSYS 2010 as well as on COBAS e 411 or an COBAS e 601 analysers.

### Example 2: S100B and sFlt-1 in healthy subjects and patients with acute inflammation

In a first step S100B and sFlt-1 levels were tested in healthy individuals and in patients with systemic inflammation. A second control group included patients with chronic artery disease and heart failure in the sFlt-1 group.

**Table 1: S 100B in healthy individuals and in patients with systemic inflammation at admission to the emergency room in comparison to healthy controls (25, 50 and 75 percentile are given)**

| S100B pg/ml | healthy | inflammation |
|---|---|---|
| 75 | 0.065 | 0.199 |
| 50 | 0.055 | 0.091 |
| 25 | 0.045 | 0.061 |

**Table 2: sFlt-1 in healthy subjects and in patients with stable heart disease and patients with systemic inflammation at admission to the emergency room (25, 50 and 75 percentile are given)**

| sFlt-1 (pg/ml) | healthy | CAD | inflammation |
|---|---|---|---|
| 75 | 71 | 124 | 193.76 |
| 50 | 52 | 94 | 128.64 |
| 25 | 42 | 75 | 101.03 |

Both tests, S100B and sFlt-1, were able to predict poor outcome (death within 30 days) as shown in Table 3

**Table 3: S100B and sFlt-1 values in patients who survived and in those who did die**

| | S 100 | died | survived | sFlT1 | died | survived |
|---|---|---|---|---|---|---|
| 75 | | 0,73 | 0,19 | | 510 | 185 |
| 50 | | 0.18 | 0.09 | | 344 | 127 |
| 25 | | 0,10 | 0.06 | | 131 | 98 |

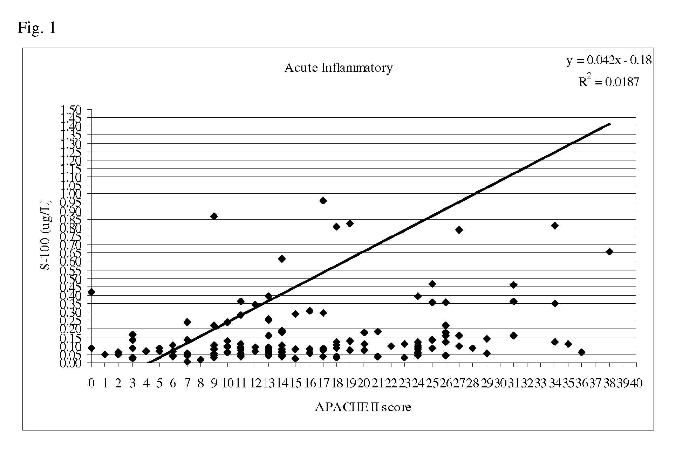

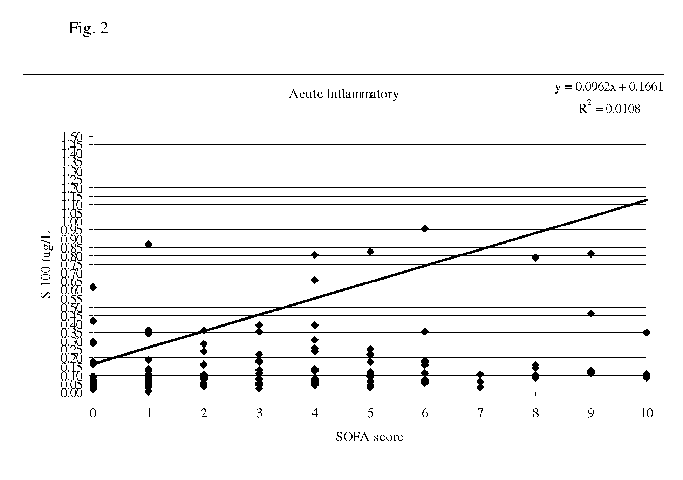

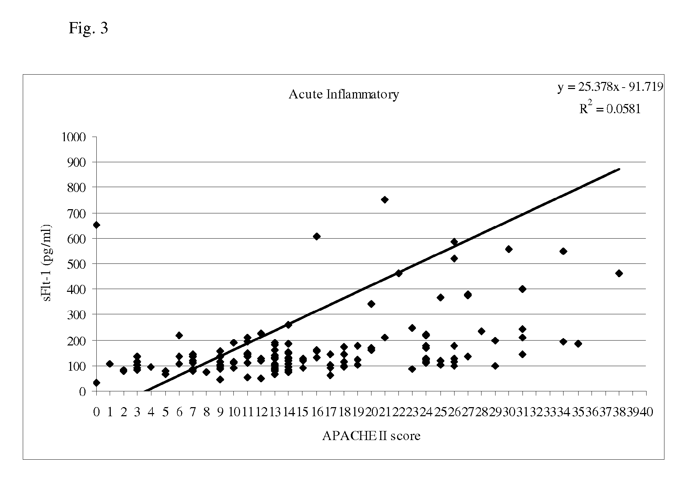

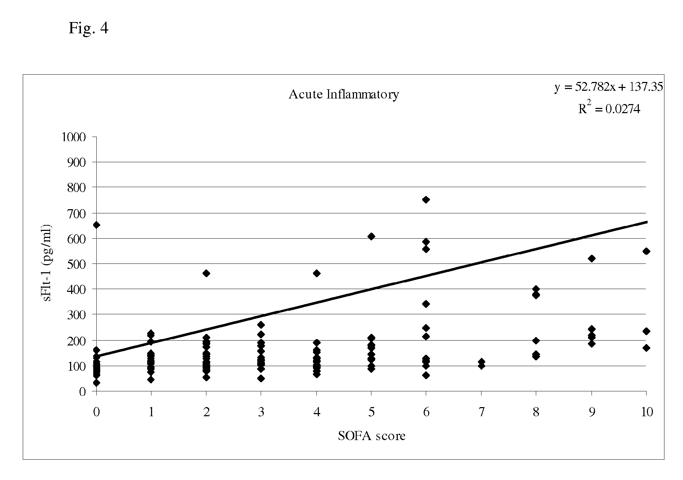

Both, S100 B and sFlT-1, were associated with the APACHE II and the SOFA Score. This is shown in Figures 1 to 4. As can be seen from the figures S100B and sFIT-1 provide information with respect to severity of the inflammatory systemic process.

### Example 3: S100B and sFlt-1 provide different diagnostic information

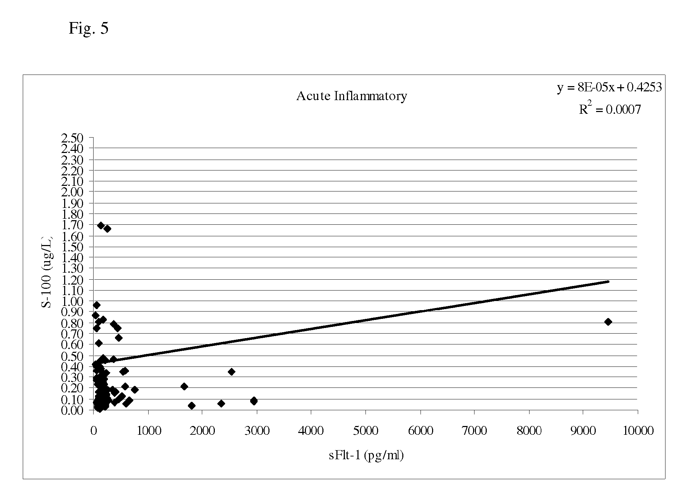

As can be seen from Figure 5, sFlt-1 and S100B did not correlate suggesting that they provide different information.

This is further supported by the associating of S 100B "low" and S 100B "High" with cardiac and inflammatory markers as shown in Tables 4 and 5

**Table 4: S100B above and below the Median (0.091) and its association with other biomarkers:**

| | Below Median | Above Median |
|---|---|---|
| S100B | 0,06 | 0,199 |
| sFlt-1 | 122 | 135 |
| NT-pro BNP | 850 | 1511 |
| Sens Troponin T | 16 | 31 |
| GDF 15 | 2627 | 5235 |

**Table 5: sFlt-1 above and below Median (128,6) and its association with other biomarkers**

| | | |
|---|---|---|
| | Below Median | Above Median |
| sFlt-1 | 100 | 193 |
| S100B | 0,082 | 0.107 |
| NT-pro BNP | 574 | 2833 |
| Sens Troponin T | 14 | 31 |
| GDF 15 | 2660 | 5975 |

As shown S100B and sFlt-1 are not interrelated, both markers are linked to inflammation (GDF 15), sFlt-1 is, however, strongly correlated with cardiac function (NT-pro BNP).

This is supported by individual cases as shown below:
In a fist series of cases, cases are presented with increased S100B levels but sFlt-1 levels which are not increased (cerebral damage without ischemia):

| **Case** | **Time Point** | **sFlt-1 pg/ml** | **S100B µg/l** |
|---|---|---|---|
| **41** | **Admission** | **91.4** | **0.291** |
| **41** | **6 hours** | | |
| **41** | **24 hours** | **70.8** | **0.236** |
| **41** | **72 hours** | **89.7** | **0.329** |
| **103** | **Admission** | **112.3** | **0.445** |
| **103** | **6 hours** | **98.5** | **0.258** |
| **103** | **24 hours** | | |
| **103** | **72 hours** | | |
| **161** | **Admission** | **88.7** | **0.220** |
| **161** | **6 hours** | | |
| **161** | **24 hours** | **51.6** | **0.299** |
| **161** | **72 hours** | **54.5** | **0.317** |
| **153** | **Admission** | **46.4** | **0.867** |
| **153** | **6 hours** | | |
| **153** | **24 hours** | **36.9** | **0.508** |
| **153** | **72 hours** | **40.4** | **0.373** |

In a second series of cases with increased S100B and increased sFlt-1 are presented indicating cerebral damage and ischemia (It shall be noted, however, that cerebral damage is not strictly related to ischemia):

| **Case** | **Time Point** | **sFlt-1 pg/ml** | **S100B µg/l** |
|---|---|---|---|
| **5** | **Admission** | **2347.0** | **0.059** |
| **5** | **6 hours** | **588.1** | **0.467** |
| **5** | **24 hours** | **527.1** | **0.268** |
| **5** | **72 hours** | **190.0** | **0.067** |
| **7** | **Admission** | **343.9** | **0.181** |
| **7** | **6 hours** | | |
| **7** | **24 hours** | **2691.3** | **8.142** |
| **7** | **72 hours** | | |
| **56** | **Admission** | **127.2** | **0.176** |
| **56** | **6 hours** | **97.0** | **0.128** |
| **56** | **24 hours** | **76.2** | **0.078** |
| **56** | **72 hours** | **67.7** | **0.054** |
| **59** | **Admission** | **103.9** | **0.084** |
| **59** | **6 hours** | | |
| **59** | **24 hours** | **65.9** | **0.130** |
| **59** | **72 hours** | **83.2** | **0.080** |
| **61** | **Admission** | **369.7** | **0.465** |
| **61** | **6 hours** | **492.5** | **0.603** |
| **61** | **24 hours** | **172.7** | **0.210** |
| **61** | **72 hours** | **420.9** | **0.102** |
| **159** | **Admission** | **62.4** | **0.960** |
| **159** | **6 hours** | | |
| **159** | **24 hours** | **193.3** | **4.130** |
| **159** | **72 hours** | **32.7** | **0.944** |
| **169** | **Admission** | **2533.2** | **0.354** |
| **169** | **6 hours** | **338.0** | **0.650** |
| **169** | **24 hours** | **235.7** | **0.517** |
| **169** | **72 hours** | **138.2** | **0.249** |

In a third series cases are presented having evidence for ischemia (increased sFlt-1 levels) but no evidence of cerebral damage:

| **Case** | **Time Point** | **sFlt-1 pg/ml** | **S100B µg/l** |
|---|---|---|---|
| **22** | **Admission** | **136.0** | **0.030** |
| **22** | **6 hours** | **95.1** | **0.025** |
| **22** | **24 hours** | **89.2** | **0.046** |
| **22** | **72 hours** | **93.2** | **0.030** |
| **92** | **Admission** | **607.6** | **0.062** |
| **92** | **6 hours** | | |
| **92** | **24 hours** | **162.5** | **0.025** |
| **92** | **72 hours** | **143.5** | **0.057** |
| **129** | **Admission** | **117.9** | **0.052** |
| **129** | **6 hours** | **496.1** | **0.058** |
| **129** | **24 hours** | | |
| **129** | **72 hours** | | |

Further cases (fourth series) show neither evidence of significant ischemia nor evidence of significant cerebral damage:

| **Case** | **Time Point** | **sFlt-1 pg/ml** | **S100B µg/ml** |
|---|---|---|---|
| **184** | **Admission** | **75.2** | **0.062** |
| **184** | **6 hours** | | |
| **184** | **24 hours** | **96.4** | **0.081** |
| **184** | **72 hours** | **103.3** | **0.091** |
| **200** | **Admission** | **90.1** | **0.039** |
| **200** | **6 hours** | | |
| **200** | **24 hours** | **100.4** | **0.043** |
| **200** | **72 hours** | **85.6** | **0.107** |

sFlt-1 and S100B provide different information in patients suspected of having acute inflammation. As can be seen sFlt-1 increase can occur without elevation of S100B and vice versa. In addition ischemia can occur in association with S100B elevation.

The data provide the following diagnostic information:
Evidence of ischemia and or cerebral damage using appropriate cut off levels taken from control groups (normal subjects and/or patients with heart failure). Cerebral damage can be identified by S 100B levels and said damage can be classified as involving ischemia or not based on increased levels of sFlt-1 which occur as a consequence of ischemia in ischemic cerebral damage. The aforementioned diagnostic methods, thus, allow for differentiation of cerebral damage as ischemic or non-ischemic cerebral damage. Based on this differentiation, therapeutic needs can be determined, such as, e.g., a need for urgent circulatory support, and suitable therapies can be immediately applied.

## Claims

1. A method for diagnosing in a subject exhibiting a symptom of acute inflammation whether a cerebral damage is an ischemic cerebral damage or a non-ischemic cerebral damage comprising:
a) determining the amount of the biomarkers S100B and sFlt-1 in a sample of said subject; and
b) comparing said amount to reference amounts whereby it is diagnosed whether the cerebral damage is an ischemic cerebral damage or a non-ischemic cerebral damage.

2. The method of claim 1, wherein said reference amount is derived from a subject or a group of subjects known to suffer from non-ischemic cerebral damage and wherein an essentially identical or increased amount of the biomarker S100B in combination with an essentially identical or decreased amount for the biomarker sFlt-1 is indicative for a subject having a non-ischemic cerebral damage and wherein an essentially identical or increased amount of the biomarker S 100B in combination with an increased amount for the biomarker sFlt-1 is indicative for a subject having a ischemic cerebral damage.

3. The method of claim 1, wherein said reference amount is derived from a subject or a group of subjects known to suffer from ischemic cerebral damage and wherein an essentially identical or increased amount of the biomarker S100B in combination with an essentially identical or increased amount for the biomarker sFlt-1 is indicative for a subject having a ischemic cerebral damage and wherein an essentially identical or increased amount of the biomarker S 100B in combination with a decreased amount for the biomarker sFlt-1 is indicative for a subject having a non-ischemic cerebral damage.

4. The method of any one of claims 1 to 3, wherein said non-ischemic cerebral damage comprises damage of the blood brain barrier.

5. The method of any one of claims 1 to 4, wherein said subject does or did not suffer from trauma and/or cardiovascular diseases.

6. The method of any one of claims 1 to 5, wherein said method further comprises recommending a therapy for the cerebral damage.

7. The method of claim 6, wherein said therapy of the cerebral damage is a circulation stabilizing therapy if the cerebral damage is ischemic cerebral damage or wherein said therapy of the cerebral damage is a detoxification therapy if the cerebral damage is non-ischemic cerebral damage.

8. A method for diagnosing whether a subject exhibiting a symptom of acute inflammation is at increased risk of mortality comprising:
a) determining the amount of the biomarker S100B in a sample of said subject; and
b) comparing said amount to a reference amount whereby it is diagnosed whether the subject is at increased risk of mortality, or not.

9. The method of claim 8, wherein said reference amount is derived from a subject or a group of subjects exhibiting a symptom of acute inflammation which is known to be at increased risk of mortality and wherein an essentially identical or increased amount of said S100B is indicative for a subject being at increased risk of mortality and wherein a decreased amount of said S 100B is indicative for a subject being at normal risk of mortality.

10. The method of claim 8, wherein said reference amount is derived from a subject or a group of subjects exhibiting a symptom of acute inflammation which is known to be at normal risk of mortality and wherein an essentially identical or decreased amount of said S 100B is indicative for a subject being at normal risk of mortality and wherein a increased amount of said S 100B is indicative for a subject being at increased risk of mortality.

11. The method of any one of claims 1 to 10, wherein TGFβ-1 is determined instead of S100B.

12. Use of the biomarker S100B and sFlt-1 or detection agents which specifically bind thereto in a sample of a subject exhibiting a symptom of acute inflammation for diagnosing whether a cerebral damage is an ischemic cerebral damage or a non-ischemic cerebral damage.

13. Use of the biomarker S 100B or a detection agent that specifically binds thereto in a sample of a subject exhibiting a symptom of acute inflammation for diagnosing whether a subject exhibiting a symptom of acute inflammation is at increased risk of mortality.

14. A device adapted for carrying out the method of any one of claims 1 to 7 comprising
a) an analyzing unit comprising a detection agent which specifically binds to S100B and detection agents which specifically bind to sFlt-1, said unit being adapted for determining the amount of S100B and the amounts of sFlt-1 in a sample of a subject exhibiting a symptom of acute inflammation; and
b) an evaluation unit for comparing the determined amounts with reference amounts whereby it can be diagnosed whether a cerebral damage is an ischemic cerebral damage or a non-ischemic cerebral damage, said unit comprising a database with reference amount values derived from a subject as defined in claim 2 or 3 and a computer-implemented algorithm for carrying out a comparison as specified in claim 2 or 3.

15. A device adapted for carrying out the method of any one of claims 8 to 10 comprising
a) an analyzing unit comprising a detection agent which specifically binds to S100B, said unit being adapted for determining the amount of S100B in a sample of a subject exhibiting a symptom of acute inflammation; and
b) an evaluation unit for comparing the determined amounts with reference amounts whereby it can be diagnosed whether a subject exhibiting a symptom of acute inflammation is at increased risk of mortality, said unit comprising a database with reference amount values derived from a subject as defined in claim 9 or 10 and a computer-implemented algorithm for carrying out a comparison as specified in claim 9 or 10.

16. A kit adapted for carrying out the method of any one of claims 1 to 7 comprising detection agents for the biomarker S100B and sFlt-1 as well as instructions for carrying out the said method.

17. A kit adapted for carrying out the method of any one of claims 8 to 10 comprising a detection agent for the biomarker S 100B as well as instructions for carrying out the said method.
